# EUROPÄISCHE PATENTSCHRIFT

(11) **EP 2 906 160 B1**
(45) Veröffentlichungstag und Bekanntmachung des Hinweises auf die Patenterteilung: **21.06.2017**
(21) Anmeldenummer: 12790392.0
(22) Anmeldetag: 15.10.2012
(51) Int. Cl.: A61F 11/00, A61F 7/12

(54) **OHRKÜHLVORRICHTUNG**
EAR COOLING DEVICE
DISPOSITIF DE REFROIDISSEMENT DE L'OREILLE

(43) Veröffentlichungstag der Anmeldung: 19.08.2015
(73) Patentinhaber: AUROX GmbH, 8010 Graz (AT)
(72) Erfinder: Schöggler, Christoph, 8142 Wundschuh (AT)
(74) Vertreter: Wirnsberger, Gernot
(86) Internationale Anmeldenummer: PCT/AT2012/050163
(87) Internationale Veröffentlichungsnummer: WO 2014/059454

(56) Entgegenhaltungen:
- DE-A1- 2 323 444
- DE-U1-202006 019 587
- US-A1- 2003 229 336
- US-A1- 2010 198 282
- US-A1- 2011 313 498

## Beschreibung

Die Erfindung betrifft eine am Ohr tragbare, mobile Vorrichtung zum Kühlen eines Außenohrs zur Unterstützung des Heilvorganges bei Gehörgangsentzündungen, umfassend einen an einem Gehörgang, insbesondere einem Gehörgang eines Menschen, anlegbaren und vorzugsweise in diesen zumindest teilweise einführbaren Ohrstöpsel und eine Kühleinrichtung, mit welcher der Ohrstöpsel und/oder im Gehörgang befindliche Luft kühlbar ist.

Bei der Behandlung verschiedener Krankheiten eines menschlichen Ohrs kann es erforderlich sein, das Ohr zu kühlen oder zu erwärmen. Hierfür werden in der Regel Flüssigkeiten, insbesondere Wasser, eingesetzt, die auf eine vorbestimmte Temperatur gebracht werden. In diesem Zusammenhang ist beispielsweise aus der JP 2006230761 A eine Vorrichtung bekannt geworden, bei der Wasser aus einem Wassertank mittels Peltierelementen auf eine vorgegebene Temperatur gebracht wird, um die Rückseite eines menschlichen Ohrs zu erwärmen.

Neben der Beaufschlagung eines menschlichen Ohrs mit einer Flüssigkeit zum Zwecke einer Heilung einer Krankheit kann auch aus anderen Gründen eine Temperaturbehandlung eines Ohrs vorgesehen sein, beispielsweise zur Prüfung der Leistungsfähigkeit und allfälliger pathologischer Veränderungen eines menschlichen Gleichgewichtsorgans. In diesem Zusammenhang ist aus der DE 23 23 444 A1 eine Vorrichtung bekannt geworden, bei welcher ein Peltierelement auf einer Seite mit einem Metallzapfen aus Kupfer in Verbindung steht, der in ein menschliches Ohr einführbar ist. Auf der gegenüberliegenden Seite ist das Peltierelement mit einer Kühleinrichtung verbunden, welche einen Wasserkreislauf aufweist. Durch Steuerung des Peltierelementes ist es möglich, den Metallzapfen auf eine bestimmte Temperatur zu bringen und damit ein Gleichgewichtsorgan zu erregen. Auf der anderen Seite wird Wärme durch das Wasser abgeführt. Die Vorrichtung kann transportabel sein, wenn die Wasserkühlung als Umlaufkühlung konstruiert ist.

Bei Patienten mit Gehörgangsentzündung (otitis externa) kommt speziell eine Kühlung des Außenohrs zur Anwendung. Otitis externa ist eine bakterielle, pilzbedingte Entzündung des äußeren Gehörgangs, durch die in weiterer Folge auch das Trommelfell betroffen sein kann. Von Ärzten wird neben einer medikamentösen Therapie insbesondere auch eine Kühlung sowie Trockenlegung des Außenohrs empfohlen, um eine rasche Linderung der Schmerzen und Genesung zu erreichen.

Ohrenentzündungen treten relativ häufig auf und sind dementsprechend auch häufig zu behandeln. Für die empfohlene Kühlung und gegebenenfalls Trockenlegung eines Ohrs durch den Patienten selbst sind gegenwärtig keine brauchbaren Vorrichtungen verfügbar. Die bekannten Vorrichtungen sind zumeist konstruktiv aufwendig und daher nicht transportabel. Dies bedingt, dass ein Patient zur Behandlung stets ein Krankenhaus oder eine andere medizinische Anstalt aufsuchen muss. US 2010/198282 A1, US 2011/313498 A1, DE 23 23 444 A1 und DE 20 2006 019587 U1 zeigen Vorrichtungen zum Kühlen des Außenohrs. US 2003/229336 A1 zeigt eine Leitung zur Zuführung eines Arzneimittels.

Aufgabe der Erfindung ist es, eine Vorrichtung anzugeben, mit welcher ein äußerer Gehörgang eines Ohrs mit geringem apparativen Aufwand kühlbar ist und wobei gleichzeitig das Ohr vor unerwünschter Feuchtigkeit und Luftzug geschützt ist.

Diese Aufgabe wird gelöst, wenn bei einer Vorrichtung der eingangs genannten Art die Kühleinrichtung ohne flüssiges Wärmetransportmittel ausgebildet ist und ein Peltierelement umfasst, dessen Innenseite mit dem Ohrstöpsel in Verbindung steht, um diesen und/oder im Gehörgang befindliche Luft zu kühlen, und über dessen Außenseite Wärme abführbar ist. Erfindungsgemäß ist kein flüssiges Wärmetransportmittel vorgesehen, sondern erfolgt ein vorzugsweise direktes Abführen von Wärme an die Luft über Abstrahlung an der Außenseite des Peltierelementes. Dabei wird die Erkenntnis ausgenutzt, dass für eine Kühlung des Außenohrs zur Unterstützung eines Heilvorganges bei Gehörgangsentzündungen nur eine geringe Temperatursenkung von wenigen Grad erforderlich ist. Größere Temperatursenkungen sind auch gar nicht erwünscht, weil dadurch der Gleichgewichtssinn gestört werden kann und darüber hinaus beim Patienten Übelkeit hervorgerufen wird. Weil für die beabsichtigten Heilzwecke, insbesondere die Behandlung von Gehörgangsentzündungen, eine geringe Kühlung bereits völlig ausreichend ist, kann beim Betrieb des Peltierelementes auf eine konstruktiv aufwendige Wasserkühlung verzichtet werden, weshalb die erfindungsgemäße Vorrichtung insgesamt mit geringen Mitteln bzw. apparativ einfach zu realisieren ist.

Für die gewünschte Kühlung ist der Ohrstöpsel am Gehörgang anlegbar, was ausreichend sein kann. Günstig ist es zumeist, dass der Ohrstöpsel zumindest teilweise in den Gehörgang einführbar ist, um die Kühlwirkung effizient am Entzündungsort wirksam werden zu lassen. Durch vorsichtiges Einführen in den Gehörgang lässt sich ein endseitiger Bereich des Ohrstöpsels mit einer entzündeten Stelle in Kontakt bringen, ohne dass die entzündete Stelle gereizt wird.

Darüber hinaus sorgt der vorgesehene Ohrstöpsel dafür, dass der Gehörgang während der Behandlung gegenüber der Umwelt zumindest weitgehend abgeschlossen sein kann bzw. kaum Kontakt zur Umwelt hat, womit ein Eindringen von unerwünschter Feuchtigkeit oder ein ungewollter Luftzug im Gehörgang vermieden wird.

Als Materialien für den Ohrstöpsel kommen erfindungsgemäß Metalle infrage. Als Metalle können beispielsweise Aluminium oder Titan sowie Legierungen mit diesen Metallen als Hauptbestandteile Anwendung finden. Möglich ist auch eine Ausbildung des Ohrstöpsels aus einem Kunststoff, insbesondere einem Polymer wie Polyoxymethylen. Dabei ist der Ohrstöpsel bevorzugt teilweise oder ganz aus einem elastischen Kunststoff gebildet. Dies ermöglicht es, dass der Ohrstöpsel beim Einführen in das Ohr angepasst wird, sodass bei der nachfolgenden Behandlung ein gewünschter Abschluss des Gehörganges gegeben ist.

Der Ohrstöpsel kann mit einem abnehmbaren Aufsatz ausgebildet sein. Der Aufsatz, der sich an einem dem Ohr zugewendeten Ende des Ohrstöpsels befindet, kann dann nach Gebrauch bzw. nach einer Behandlung abgenommen und durch einen neuen Aufsatz ersetzt werden. Dadurch kann ein hygienischer reiner Aufsatz für jede einzelne Behandlung verwendet werden.

Vorgesehen sein kann auch, dass der Ohrstöpsel zumindest teilweise beschichtet ist. Eine Beschichtung kann aus verschiedenen Gründen vorgesehen sein. Eine Beschichtung mit Silicon ist beispielsweise dann zweckmäßig, wenn der Ohrstöpsel aus einem Metall besteht. Die außenseitige Beschichtung mit Silicon verleiht dem Ohrstöpsel dann eine gewünschte Elastizität im Außenbereich, sodass dieser trotz an sich starrer Fertigung aus einem Metall in ein Ohr bzw. einen Gehörkanal einführbar und an diesen anpassbar ist. Eine Beschichtung kann aber auch vorgesehen sein, um eine antibakterielle Wirkung zu verstärken. Hierfür wird z. B. eine Beschichtung aus Silber eingesetzt. Möglich ist es auch im Rahmen der Erfindung, dass eine Beschichtung eingesetzt wird, die im Laufe der Zeit langsam Silberionen freisetzt und somit dauerhaft antibakteriell wirkt. Dabei kann es ausreichend sein, dass der Ohrstöpsel nur in bestimmten Bereichen mit der antibakteriell wirkenden Beschichtung versehen ist.

Der Ohrstöpsel ist grundsätzlich so geformt, dass dieser an einem ersten Ende, das in den Gehörgang einführbar ist, eine dichte bzw. durchgehende Oberfläche bildet. Der Ohrstöpsel als solcher wird durch das Peltierelement gekühlt, sodass die gewünschte Kühlwirkung erzielt wird. Möglich ist es allerdings auch, dass der Ohrstöpsel an dem ersten Ende eine Öffnung aufweist. Bei dieser Variante kann im Gehörgang befindliche Luft in den Ohrstöpsel eintreten und dort gekühlt werden. Die geringe Zirkulation ist dann ausreichend, um die im Ohr befindliche Luft und damit letztlich auch den Gehörgang zu kühlen. Erfindungsgemäß ist das Peltierelement in einem endseitigen Bereich des Ohrstöpsels und innerhalb desselben angeordnet. Dadurch ist ein einfacher und kompakter Aufbau gegeben. Insbesondere ist es dadurch auch möglich, dass der Ohrstöpsel das Peltierelement weitgehend umhüllt, sodass lediglich die Außenseite des Peltierelementes, die für die Wärmeabfuhr vorgesehen ist, zugänglich ist. Diese Außenseite kann zudem beispielsweise mit einem die Kühlwirkung verstärkenden Element ausgebildet sein, z. B. einem gerippten Metallblättchen, das mit dem Peltierelement verbunden ist und über dessen Oberfläche die Wärme abgegeben wird. Von Vorteil ist dabei, dass die Vorrichtung durch den Patienten nicht nur einfach handhabbar, sondern durch diesen auch kaum beschädigbar ist.

Zum Betrieb des Peltierelementes ist zweckmäßigerweise eine erste Leitung bzw. ein Kabel vorgesehen, mit dem das Peltierelement zur Stromversorgung in Verbindung steht. Das Kabel weist üblicherweise an einem Ende einen Stecker auf, mit dem es an eine Stromversorgungsquelle anschließbar ist. Besonders günstig ist es, dass das Kabel einen endseitigen Stecker aufweist, der an einem Mobiltelefon ansteckbar ist. Dadurch kann unterwegs auf einfache Weise eine Stromversorgung der erfindungsgemäßen Vorrichtung sichergestellt werden.

Zweckmäßig ist es in der Regel auch, dass eine Versorgungs- und Steuereinheit vorgesehen ist, die mit dem Peltierelement in Verbindung steht und mit der das Peltierelement regelbar ist. Die Versorgungs- und Steuereinheit kann an beliebiger Position vorgesehen sein, ist aber zweckmäßigerweise zwischen Stromquelle und der erfindungsgemäßen Vorrichtung angeordnet. Dadurch können Kühlabläufe beliebig gestaltet werden. Beispielsweise ist es möglich, eine Intervallkühlung oder auch eine pulsierende Kühlung einzustellen. Darüber hinaus erlaubt die Versorgungs- und Steuereinheit die Vorgabe bestimmter Maximalwerte für die Temperatur, sodass ein Temperaturbereich eingestellt werden kann, innerhalb dessen die Behandlung erfolgen soll. Dadurch kann beispielsweise ein unerwünschtes Unterschreiten einer Minimaltemperatur vermieden werden, unterhalb welcher es aufgrund einer Störung des Gleichgewichtssinnes zu Übelkeit kommen könnte. In diesem Zusammenhang kann auch ein Display zur Temperaturanzeige vorgesehen sein. Zur Ausgabe diverser Parameter kann alternativ oder darüber hinaus auch eine akustische Ausgabeeinrichtung integriert sein.

Die Versorgungs- und Steuereinheit kann einen Akkumulator und ein Bedienelement zur Temperatureinstellung sowie einen Gleichrichter zur Gleichstromerzeugung umfassen.

Wenn eine Stromversorgung an sich bereits durch ein Mobiltelefon sichergestellt wird, kann die Versorgungs- und Steuereinheit auch im Ohrstöpsel integriert sein. In diesem Fall besteht ein Set zum Kühlen eines Gehörganges eines Ohrs aus einer erfindungsgemäßen Vorrichtung samt Kabel und einem Mobiltelefon. Die Versorgungs- und Steuereinheit sorgt dann dafür, dass zum Betrieb des Peltierelementes ein erforderlicher Gleichstrom bereitgestellt wird. Der Strom wird vom Mobiltelefon bereitgestellt. Vorteil dieser Variante ist, dass über das Bedienfeld des Mobiltelefons bzw. eine entsprechende Applikation auch die Kühlung des Ohrstöpsels eingestellt werden kann.

Wenn eine Temperatur genau geregelt werden soll, ist es zweckmäßig, wenn im Ohrstöpsel ein Temperatursensor angeordnet ist. Der Temperatursensor ist dabei bevorzugt im Bereich des ersten, dem Gehörgang zugewendeten Endes des Ohrstöpsels angeordnet.

Insbesondere bei der erwähnten Kombination der erfindungsgemäßen Vorrichtung mit einem Mobiltelefon ist es zweckmäßig, dass im Ohrstöpsel ein Lautsprecher integriert ist. Somit kann während der Behandlung eines Patienten auch Musik gehört oder Telefonate geführt werden.

Der Ohrstöpsel ist grundsätzlich so ausgebildet, dass dieser für sich alleine bereits fest im Ohr hält. Es kann jedoch auch vorgesehen sein, dass am Ohrstöpsel eine Halterung anschließt, mit welcher die Vorrichtung am Ohr befestigbar ist. Dadurch kann ein zusätzlicher Halt bzw. eine besonders feste Fixierung am Ohr erreicht werden. Als Nebenaspekt ergibt sich dabei auch, dass bei Bedarf auch die Halterung kühlbar gestaltet sein kann, sodass auch Außenbereiche des Ohrs kühlbar sind.

Für Behandlungen, die zusätzlich auch eine Zufuhr von Arzneimittel und/oder Desinfektionsmittel erfordern, bietet es sich an, zusätzlich eine Leitung für die Zuführung eines Arzneimittels oder Desinfektionsmittels vorzusehen, die an einer äußeren Oberfläche des Ohrstöpsels endet. Dies erlaubt es bei Behandlung neben der Kühlung gleichzeitig auch die Heilung fördernde Substanzen zuzuführen.

Weitere Merkmale, Vorteile und Wirkungen der Erfindung ergeben sich aus den nachfolgend dargestellten Ausführungsbeispielen. In den Zeichnungen, auf welche dabei Bezug genommen wird, zeigen:
Fig. 1 eine Vorrichtung;
Fig. 2 die Anbringung einer Vorrichtung in einem Ohr bzw. Gehörgang;
Fig. 3 eine weitere Vorrichtung;
Fig. 4 eine Vorrichtung mit einer Versorgungs- und Steuereinheit;
Fig. 5 eine weitere Variante einer Vorrichtung;
Fig. 6 eine Vorrichtung mit mehreren Leitungen;
Fig. 7 eine erfindungsgemäße Vorrichtung mit einem Aufsatz in Draufsicht;
Fig. 8 die Vorrichtung gemäß Fig. 7 in einer stirnseitigen Ansicht;
Fig. 9 die Vorrichtung gemäß Fig. 7 in einem Querschnitt entlang der Linie IX-IX in Fig. 7;
Fig. 10 ein Detail der Vorrichtung gemäß Fig. 9;
Fig. 11 eine weitere Variante einer erfindungsmäßen Vorrichtung.

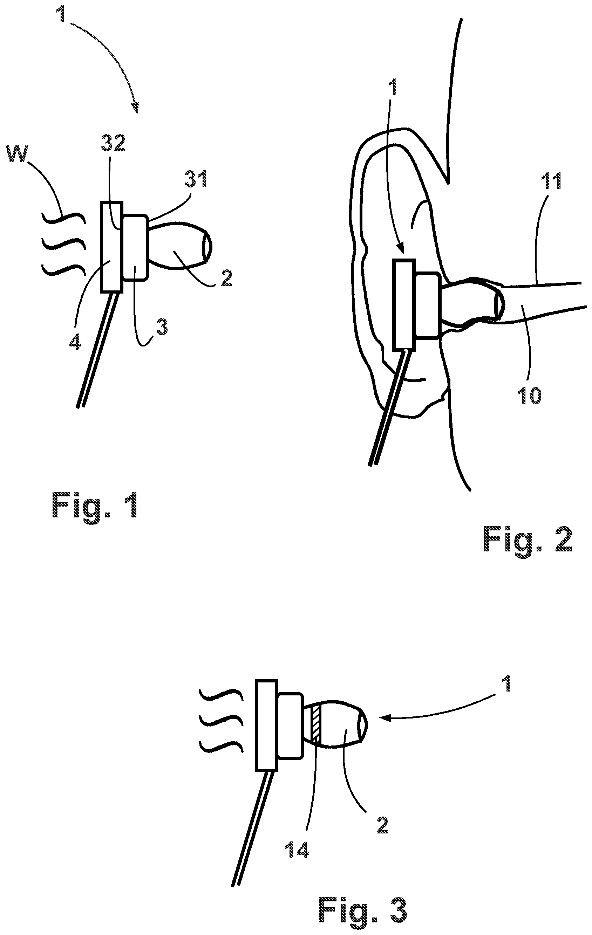

In Fig. 1 ist eine Vorrichtung 1 dargestellt. Die Vorrichtung 1 umfasst einen Ohrstöpsel 2 sowie ein Peltierelement 3 und einen Kühlkörper 4. Das Peltierelement 3 steht an einer Innenseite 31 mit dem Ohrstöpsel 2 in Kontakt. An einer Außenseite 32 steht das Peltierelement 3 mit einem Kühlkörper 4 in Kontakt. Der Kühlkörper 4 kann vorgesehen sein, dies ist jedoch nicht zwingend. Darüber hinaus ist eine Leitung 5 vorgesehen, welche durch den Kühlkörper 4 führt und über welche dem Peltierelement 3 Strom zum Betrieb desselben zugeführt wird.

Der Ohrstöpsel 2 kann aus einem beliebigen Material gebildet sein. Üblicherweise ist der Ohrstöpsel 2 aus einem Metall wie Aluminium oder Titan oder Legierungen dieser Metalle gebildet. Insbesondere Titan oder Titanlegierungen können vorgesehen sein, weil diese biokompatibel sind bzw. keine allergischen Reaktionen hervorrufen. Besonders bevorzugt ist es jedoch, dass der Ohrstöpsel 2 aus einem elastischen Kunststoff besteht. Hierfür kommt beispielsweise Polyoxymethylen infrage. Der Ohrstöpsel 2 ist dann mit leichtem Druck elastisch verformbar und kann sich einem Gehörgang 10 bzw. einer Gehörgangswand 11 anpassen, wie dies in Fig. 2 schematisch dargestellt ist. Allgemein ist es günstig, dass der Ohrstöpsel 2 aus einem anpassungsfähigen und hautverträglichen Material besteht. Insbesondere der Einsatz von Metallen kann es sich auch anbieten, eine zusätzliche Beschichtung aus einem elastischen Material anzubringen, beispielsweise Silicon, damit die gewünschte Elastizität und damit Anpassungsfähigkeit zumindest im Außenbereich des Ohrstöpsels 2 gegeben ist.

Wie in Fig. 1 und 2 ersichtlich ist, kommt eine Vorrichtung 1 gänzlich ohne flüssiges Kühlmedium bzw. Wärmetransportmittel aus. Dadurch ist die Vorrichtung 1 kompakt herstellbar, mobil und insbesondere auch leicht transportabel. Dies ermöglicht es einen Patienten jederzeit, eine vorhandene Stromversorgung vorausgesetzt, die Vorrichtung 1 einzusetzen. Dadurch ist eine große Flexibilität bei akut auftretenden Schmerzen gegeben. Durch den elastischen Ohrstöpsel 2 ist gleichzeitig ein im Wesentlichen dichter Abschluss des Gehörganges 10 gegeben, sodass bei einer Behandlung eines akuten Schmerzes auch kein Luftzug von außen im Ohr auftritt.

Wie in Fig. 3 schematisch dargestellt, kann eine Vorrichtung 1 auch einen Lautsprecher 14 umfassen, sodass während einer Behandlung Musik gehört werden kann oder gegebenenfalls auch ein Telefonat führbar ist.

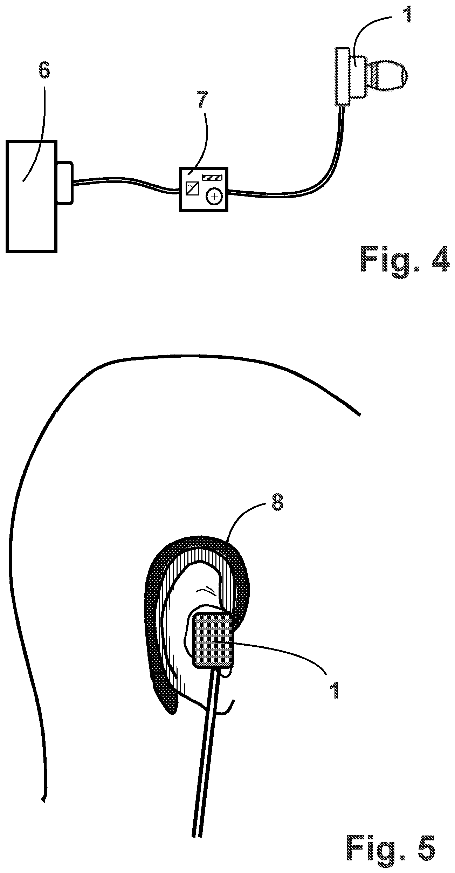

In Fig. 4 ist eine Vorrichtung 1 dargestellt, die mit einem Mobiltelefon 6 über eine Versorgungs- und Steuereinheit 7 in Verbindung steht. In diesem Fall weist der Ohrstöpsel 2 zweckmäßigerweise einen nicht dargestellten Temperatursensor auf, der beispielsweise in einem Bereich eines ersten Endes des Ohrstöpsels 2, das dem Gehörgang 10 zugewendet ist, angeordnet ist. Über eine erste Leitung 5 bzw. ein Kabel ist das Peltierelement 3 mit der Versorgungs- und Steuereinheit 7 verbunden. Analoges gilt für den integrierten, nicht dargestellten Temperatursensor. Mit der Versorgungs- und Steuereinheit 7 kann dann einerseits eine Temperatur exakt eingestellt werden. Vorzugsweise wird ein Temperaturintervall eingestellt, innerhalb dessen das Peltierelement 3 arbeiten soll. Über das Mobiltelefon 6 wird die Versorgungs- und Steuereinheit 7 mit Strom angespeist, in der Regel ein Wechselstrom. Daher ist in der Versorgungs- und Steuereinheit 7 zusätzlich ein Gleichrichter vorgesehen, um den Gleichstrom zum Betrieb des Peltierelementes 3 zu erzeugen.

Es kann auch vorgesehen sein, dass die erste Leitung 5 bzw. das Kabel mit einem endseitigen Stecker ausgebildet ist, sodass die Vorrichtung 1 unmittelbar an ein Mobiltelefon 6 oder gegebenenfalls ein anderes Endgerät wie einen MP3-Player oder einen Laptop anschließbar ist, z. B. über einen USB-Port oder eine andere standardisierte Schnittstelle. In diesem Fall ist es zweckmäßig, dass eine Einstellung der Temperatur bzw. allgemein ein Betrieb der Vorrichtung 1 über das Mobiltelefon 6 vorgenommen wird, insbesondere einer am Mobiltelefon 6 laufenden Applikation. Sofern insbesondere zur Generierung von Gleichstrom erforderlich, kann die Versorgungs- und Steuereinheit 7 im Ohrstöpsel 2 integriert sein, wie später noch erläutert werden wird.

In Fig. 5 ist eine Vorrichtung dargestellt, welche zusätzlich eine Halterung 8 umfasst. Die Halterung 8 kann zwei Funktionen haben. Zum einen dient die Halterung 8 zur noch besseren Fixierung der Vorrichtung 1 an einem menschlichen Ohr. Zum anderen kann auch vorgesehen sein, dass die Halterung 8 mitgekühlt wird, wodurch sich auch Außenbereiche des Ohrs behandeln lassen, z. B. bei Schwellungen im Außenbereich des Ohrs.

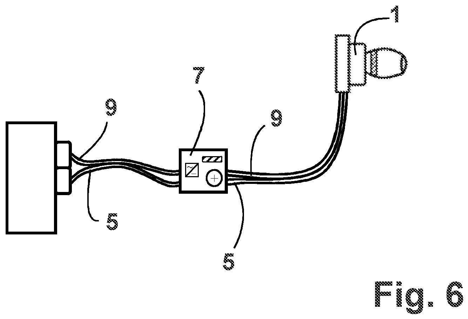

In Fig. 6 ist eine Vorrichtung 1 dargestellt, die neben der ersten Leitung 5 bzw. dem Kabel eine zweite Leitung 9 umfasst, die hohl ausgebildet ist und der Zufuhr eines Arzneimittels und/oder Desinfektionsmittels dient. Hierfür ist ein gesonderter Tank vorgesehen, über welchen geringe Mengen eines Arzneimittels zugeführt werden können.

Die zweite Leitung 9 kann aber auch anders ausgebildet sein, nämlich wie die erste Leitung 5 als Kabel. In diesem Fall kann in der ersten Leitung 5 die Spannungsversorgung untergebracht sein, während in der zweiten Leitung 9 ein Audiosignal übertragbar ist.

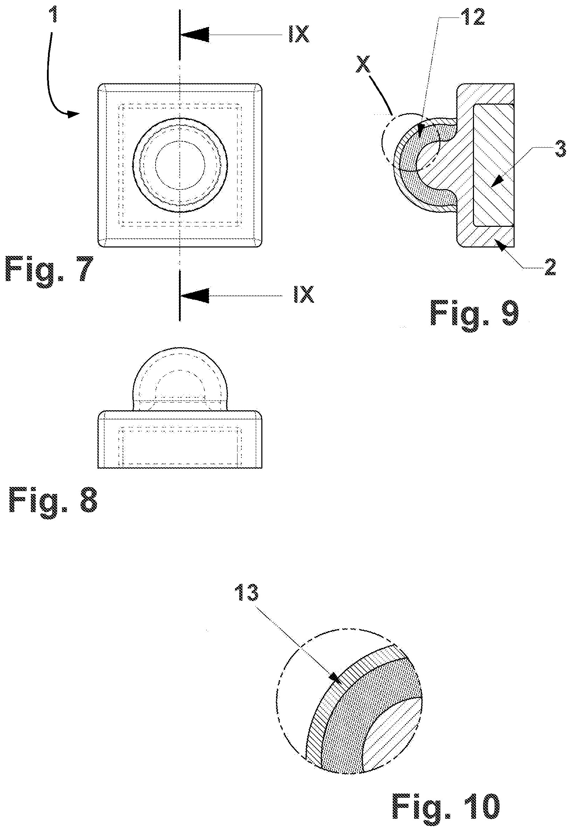

In Fig. 7 bis 10 ist eine weitere Variante einer Vorrichtung 1 dargestellt. Die Vorrichtung 1 umfasst wiederum einen Ohrstöpsel 2, der aus einem elastischen Kunststoff gebildet ist. In diesem Fall ist der Ohrstöpsel 2 mit einem 3D-Drucker aus einem Polymer gedruckt. An einem Ende weist der Ohrstöpsel 2 eine Ausnehmung auf, in welcher das Peltierelement 3 eingesetzt ist. Neben dem Peltierelement 3, das bloß stark schematisiert dargestellt ist, kann auch eine Versorgungs- und Steuereinheit 7 in den Ohrstöpsel 2 eingebettet sein. Der Ohrstöpsel 2, der elastisch verformbar ist, umfasst somit bis auf eine Stromversorgung alle zur Kühlung eines Ohrs erforderlichen Komponenten. Dabei ist von Vorteil, dass durch die Umfassung der elektronischen Bauteile durch den Ohrstöpsel 2 eine Beschädigung der Vorrichtung 1 durch einen Patienten auch bei wiederholtem Einsatz der Vorrichtung 1 kaum möglich ist. Dieser Beschädigungsschutz und somit eine Langlebigkeit des Produktes kann noch weiter verbessert werden, wenn an dem Ohr abgewendeten Ende der Ohrstöpsel 2 durch einen Kühlkörper 4 abgeschlossen ist, der nicht nur Wärme vom Peltierelement 3 verbessert abgibt, sondern auch einen mechanischen Schutz darstellt. Hierfür eignet sich ein Metallplättchen oder eine Metallplatte, die vorzugsweise mit Rippen ausgebildet sind. Darüber hinaus kann der an sich nicht zwingend erforderliche Kühlkörper 4 auch ästhetisch ansprechend ausgeformt und/oder an eine Außenseite des Ohrs angepasst sein. Erfindungsgemäß ist der Ohrstöpsel 2 gemäß Fig. 7 bis 10 aus einem Metall oder einer Legierung, insbesondere Aluminium oder einer Aluminiumlegierung, gebildet. In diesem Fall wird eine gute Wärmeleitfähigkeit vom Peltierelement 3 weg erreicht.

Bei der Ausführungsvariante gemäß Fig. 7 bis 10 kann ein Aufsatz 12 vorgesehen sein, der endseitig am Ohrstöpsel 2 aufsteckbar ist, und zwar unabhängig davon, aus welchem Material der Ohrstöpsel 2 besteht. Zu diesem Zweck ist der Aufsatz 12 mit Vorteil aus einem elastischen Material gebildet, sodass sich der endseitige Vorsprung am Ohrstöpsel 2 leicht mit dem Aufsatz 12 überziehen lässt. Der Aufsatz 12 ist auf einfache Weise austauschbar und kann durch einen neuen ersetzt werden, beispielsweise bei Verwendung der Vorrichtung 1 durch verschiedene Personen. Möglich ist es auch, dass neben universiell einsetzbaren Aufsätzen 12 spezielle Aufsätze 12 für ein linkes Ohr und ein rechtes Ohr vorgesehen sind. Wenngleich der Aufsatz 12 bevorzugt aufgesteckt wird, sind auch andere Verbindungsarten möglich, z. B. Aufschrauben, Aufschieben oder Ankletten.

Darüber hinaus können auch noch andere Komponenten im Ohrstöpsel 2 vorgesehen sein. In einer Variante ist im Ohrstöpsel 2 ein kleiner Tank vorgesehen, der ein Arzneimittel und/oder Desinfektionsmittel enthält. Der Tank kann dabei so ausgebildet sein, dass auf Druck ein Arzneimittel freigegeben wird. Der Tank kann auch in einem Aufsatz 12 vogesehen sein. Durch verschiedene Aufsätze 12 lassen sich dann unterschiedliche Arzneimittel zuführen.

Möglich ist es auch, wie insbesondere in Fig. 10 dargestellt, dass der Ohrstöpsel 2 zumindest teilweise mit einer Beschichtung versehen ist. Beispielsweise kann der Aufsatz 12 beschichtet sein. Mit einer Beschichtung kann ein Tragekomfort verbessert werden, beispielsweise mit antiallergischen Beschichtungen. Möglich ist es aber auch, antibakteriell wirksame Beschichtungen vorzusehen. Eine andere Möglichkeit der Beschichtung ist mit einem bestimmten Wirkstoff, der während der Behandlung langsam freigesetzt wird.

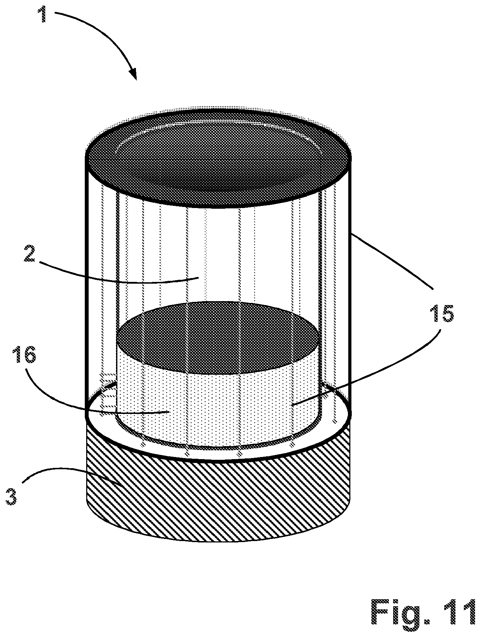

In Fig. 11 ist eine weitere Variante einer erfindungsgemäßen Vorrichtung 1 dargestellt. In diesem Fall ist wiederum das Peltierelement 3 unmittelbar mit einem flexiblen Ohrstöpsel 2 verbunden. Der Ohrstöpsel 2 besteht wiederum aus einem Polymermaterial, das allerdings von einer Vielzahl von wärmeleitenden Drähten 15, insbesondere Metalldrähten, durchzogen ist, die über einen Metallblock 16 mit dem Peltierelement 3 in Verbindung stehen. Durch die wärmeleitenden Drähte 15 ist ein Wärmetransport optimiert, sodass bei Bedarf eine besonders rasche Kühlung erreicht wird. Denkbar ist es in diesem Zusammenhang auch, ein Polymer einzusetzen, dass mit einem metallischen Pulver angereichert ist. In diesem Fall kann unter Umständen auf die wärmeleitenden Drähte 15 verzichtet werden.

Wenngleich die vorstehenden Ausführungsbeispiele hauptsächlich in Bezug auf eine Vorrichtung 1 zum Einsatz bei der Behandlung eines Menschen erläutert wurden, kann die Vorrichtung 1 selbstverständlich auch bei Tieren eingesetzt werden. In diesem Fall ist es empfehlenswert, dass die Vorrichtung 1 zusätzlich ein Halsband oder dergleichen umfasst, mit welchem die Vorrichtung am Körper eines zu behandelnden Tieres fixierbar ist.

In weiteren Varianten kann der Ohrstöpsel 2 gemäß einer der vorstehend erläuterten Ausführungsvarianten ausgebildet sein, zusätzlich aber auch eine Aufnahme für eine Batterie umfassen, sodass die Vorrichtung 1 ohne zusätzliche Geräte betreibbar ist. Darüber hinaus kann der Ohrstöpsel 2 insbesondere in jenen Bereichen, die in das Ohr eingeführt werden, mit einer strukturierten Oberfläche ausgebildet sein, z. B. mit Rillen, Löcher, Noppen oder dergleichen, um therapiebegleitend topische flüssige oder cremige Substanzen applizieren zu können.

Die erfindungsgemäße Vorrichtung 1 kann bei entsprechender Abwandlung auch zum Einführen in eine andere Körperöffnung wie eine Nase, Vagina oder einen Anus ausgebildet sein, wobei der Ohrenstöpsel 2 dann bei sonst gleichbleibenden Merkmalen als entsprechend adaptierter Nasen-, Vagina- oder Anusstöpsel fungiert und am Körper getragen und/oder in der Körperöffnung gehalten wird.

## Patentansprüche

1. Am Ohr tragbare, mobile Vorrichtung (1) zum Kühlen eines Außenohrs zur Unterstützung des Heilvorganges bei Gehörgangsentzündungen, umfassend einen an einem Gehörgang (10), insbesondere einem Gehörgang (10) eines Menschen, anlegbaren und vorzugsweise in diesen zumindest teilweise einführbaren Ohrstöpsel (2) und eine Kühleinrichtung, mit welcher der Ohrstöpsel (2) und/oder im Gehörgang (10) befindliche Luft kühlbar ist, wobei der Ohrstöpsel (2) aus einem Metall und mit einer dichten, durchgehenden Oberfläche gebildet ist und die Kühleinrichtung ohne flüssiges Wärmetransportmittel ausgebildet ist und die Kühleinrichtung ein Peltierelement (3) umfasst, dessen Innenseite (31) mit dem Ohrstöpsel (2) in Verbindung steht, um diesen und/oder im Gehörgang (10) befindliche Luft zu kühlen, und über dessen Außenseite (32) Wärme abführbar ist, wobei das Peltierelement (3) in einem endseitigen Bereich des Ohrstöpsels (2) angeordnet ist, wobei eine Außenseite des Peltierelementes (3) zugänglich ist und eine Versorgungs- und Steuereinheit (7) vorgesehen ist, die mit dem Peltierelement (3) in Verbindung steht und mit der das Peltierelement (3) regelbar ist,
**dadurch gekennzeichnet, dass** das Peltierelement (3) innerhalb des Ohrstöpsels (2) angeordnet ist.

2. Vorrichtung (1) nach Anspruch 1, **dadurch gekennzeichnet, dass** der Ohrstöpsel (2) mit einem abnehmbaren Aufsatz (12) ausgebildet ist.

3. Vorrichtung (1) nach Anspruch 1 oder 2, **dadurch gekennzeichnet, dass** der Ohrstöpsel (2) zumindest teilweise beschichtet ist.

4. Vorrichtung (1) nach einem der Ansprüche 1 bis 3, **dadurch gekennzeichnet, dass** eine erste Leitung (5) bzw. ein Kabel vorgesehen ist, mit dem das Peltierelement (3) zur Stromversorgung desselben in Verbindung steht.

5. Vorrichtung (1) nach Anspruch 4, **dadurch gekennzeichnet, dass** das Kabel einen endseitigen Stecker aufweist, der an einem Mobiltelefon (6) ansteckbar ist.

6. Vorrichtung (1) nach Anspruch 1, **dadurch gekennzeichnet, dass** die Versorgungs- und Steuereinheit (7) einen Akkumulator und ein Bedienelement zur Temperatureinstellung sowie einen Gleichrichter zur Gleichstromerzeugung umfasst.

7. Vorrichtung (1) nach einem der Ansprüche 1 bis 6, **dadurch gekennzeichnet, dass** im Ohrstöpsel (2) ein Temperatursensor angeordnet ist.

8. Vorrichtung (1) nach einem der Ansprüche 1 bis 7, **dadurch gekennzeichnet, dass** im Ohrstöpsel (2) ein Lautsprecher (14) integriert ist.

9. Vorrichtung (1) nach einem der Ansprüche 1 bis 8, **dadurch gekennzeichnet, dass** am Ohrstöpsel (2) eine Halterung (8) anschließt, mit welcher die Vorrichtung (1) am Ohr befestigbar ist.

10. Vorrichtung (1) nach einem der Ansprüche 1 bis 9, **dadurch gekennzeichnet, dass** eine zweite Leitung (9) für die Zuführung eines Arzneimittels oder Desinfektionsmittels vorgesehen ist, die an einer äußeren Oberfläche des Ohrstöpsels (2) endet.

## Claims

1. Mobile device (1) that can be worn on the ear for cooling the outer ear in order to support the healing process in the case of ear canal inflammations, comprising an ear plug (2) which can be placed against an ear canal (10), particularly an ear canal (10) of a human being, and can preferably be at least partially inserted into said ear canal, and a cooling device by means of which the ear plug (2) and/or air located in the ear canal (10) can be cooled, wherein the ear plug (2) is made from a metal and has a sealed, continuous surface and the cooling device is embodied without a liquid means of heat transport and the cooling device comprises a Peltier element (3), the inner side (31) of which is connected to the ear plug (2) in order to cool said ear plug and/or air located in the ear canal (10), and via the outer side (32) of which heat can be dissipated, wherein the Peltier element (3) is arranged in an end area of the ear plug (2), wherein an external side of the Peltier element (3) is accessible and a supply and control unit (7) is provided which is connected to the Peltier element (3) and with which the Peltier element (3) can be controlled, **characterised in that** the Peltier element (3) is arranged inside the ear plug (2).

2. Device (1) according to claim 1, **characterized in that** the ear plug (2) is embodied with a removable attachment (12).

3. Device (1) according to claim 1 or 2, **characterised in that** the ear plug (2) is at least partially coated.

4. Device (1) according to any one of claims 1 to 3, **characterised in that** a first line (5) or a cable is provided, to which the Peltier element (3) is connected in order to supply power thereto.

5. Device (1) according to claim 4, **characterised in that** the cable comprises a plug at its end, which plug can be plugged into a mobile telephone (6).

6. Device (1) according to claim 1, **characterised in that** the supply and control unit (7) comprises a rechargeable battery and an operating element for adjusting the temperature and a rectifier for generating direct current.

7. Device (1) according to any one of claims 1 to 6, **characterised in that** a temperature sensor is arranged in the ear plug (2).

8. Device (1) according to any one of claims 1 to 7, **characterised in that** a loudspeaker (14) is arranged in the ear plug (2).

9. Device (1) according to any one of claims 1 to 8, **characterised in that** a mount (8) connects to the ear plug (2), by means of which mount the device (1) can be attached to the ear.

10. Device (1) according to any one of claims 1 to 9, **characterised in that** a second line (9) is provided for delivery of a drug or disinfectant, which line ends at an outer surface of the ear plug (2).

## Revendications

1. Dispositif mobile (1) portable à l'oreille, destiné à refroidir une oreille externe pour favoriser le processus de guérison lors d'inflammations du conduit auditif, comprenant un bouchon d'oreille (2) qui peut se poser sur le conduit auditif (10), notamment sur le conduit auditif (10) d'un individu et de préférence qui peut s'insérer au moins en partie dans celui-ci et un système de refroidissement à l'aide duquel le bouchon d'oreille (2) et/ou de l'air se trouvant dans le conduit auditif (10) peut être refroidi, le bouchon d'oreille (2) étant conçu en un métal et avec une surface continue étanche et le système de refroidissement étant conçu sans moyen de transport thermique liquide et le système de refroidissement comprenant un élément Peltier (3) dont la face interne (31) est en liaison avec le bouchon d'oreille (2), pour refroidir celui-ci ou de l'air se trouvant dans le conduit auditif (10) et par l'intermédiaire de la face extérieure (32) duquel de la chaleur peut être dissipée, l'élément Peltier (3) étant placé dans une zone d'extrémité du bouchon d'oreille (2), une face extérieure de l'élément Peltier (3) étant accessible et une unité d'alimentation et de commande (7) étant prévue, qui est en liaison avec l'élément Peltier (3) et avec laquelle l'élément Peltier (3) est régulable, **caractérisé en ce que** l'élément Peltier (3) est placé à l'intérieur du bouchon d'oreille (2).

2. Dispositif (1) selon la revendication 1, **caractérisé en ce que** le bouchon d'oreille (2) est conçu avec un embout (12) amovible.

3. Dispositif (1) selon la revendication 1 ou 2, **caractérisé en ce que** le bouchon d'oreille (2) est au moins partiellement revêtu.

4. Dispositif (1) selon l'une quelconque des revendications 1 à 3, **caractérisé en ce qu'**il est prévu un premier conduit (5) ou un câble avec lequel l'élément Peltier (3) est en liaison, pour l'alimentation électrique de celui-ci.

5. Dispositif (1) selon la revendication 4, **caractérisé en ce que** le câble comporte un connecteur d'extrémité, qui peut s'enficher dans un téléphone mobile (6).

6. Dispositif (1) selon la revendication 1, **caractérisé en ce que** l'unité d'alimentation et de commande (7) comprend un accumulateur et un élément de manipulation pour le réglage de la température, ainsi qu'un redresseur pour générer un courant continu.

7. Dispositif (1) selon l'une quelconque des revendications 1 à 6, **caractérisé en ce qu'**un capteur de température est placé dans le bouchon d'oreille (2).

8. Dispositif (1) selon l'une quelconque des revendications 1 à 7, **caractérisé en ce qu'**un haut-parleur (14) est intégré dans le bouchon d'oreille (2).

9. Dispositif (1) selon l'une quelconque des revendications 1 à 8, **caractérisé en ce que** sur le bouchon d'oreille (2) se raccorde une fixation (8) à l'aide de laquelle le dispositif (1) peut se fixer sur l'oreille.

10. Dispositif (1) selon l'une quelconque des revendications 1 à 9, **caractérisé en ce qu'**il est prévu un deuxième conduit (9) pour l'alimentation d'un médicament ou d'un désinfectant qui se termine sur une surface extérieure du bouchon d'oreille (2).
